# EUROPEAN PATENT SPECIFICATION

(11) **EP 2 692 415 B1**
(45) Date of publication and mention of the grant of the patent: **15.10.2014**
(21) Application number: 12460054.5
(22) Date of filing: 28.08.2012
(51) Int. Cl.: B01D 53/26, C10L 3/08, C10L 3/10, F23J 15/02, F23L 7/00

(54) **The manner of and the device for increasing biogas net calorific value**
Die Methode und die Anlage zur Energieeffizienzsteigerung vom Biogas
La méthode et l'appareil pour augmenter le pouvoir calorifique du biogaz

(30) Priority: 31.07.2012 PL 40020112
(43) Date of publication of application: 05.02.2014
(73) Proprietor: Instytut Tele-I Radiotechniczny, 03-450 Warszawa (PL)
(72) Inventor: Opalinska, Teresa, 92-763 Warszawa (PL); Wiech, Maciej, 01-227 Warszawa (PL); Maciejak, Andrzej, 01-909 Warszawa (PL); Majdak, Malgorzata, 21-400 Lukow (PL); Wnek, Bartlomiej, 96-500 Sochaczew (PL); Krysinskl, Tomasz, 01-002 Warszawa (PL)
(74) Representative: Zatorska, Zofia

(56) References cited:
- EP-A1- 2 169 035
- WO-A1-2012/006729
- DE-A1- 4 220 865

## Description

The subject of this invention is the manner of and the device for increasing the net calorific value of biogas, used particularly for gas engines.

Biogas is a combustible gas generated as a result of anaerobic fermentation of organic compounds, generally called biomass, such as: liquid manure, farm and food industry waste, excrement, and animal droppings, and partly also as a result of their putrefactive disintegration, in a biogas plant.

Unrefined biogas is composed of 70 - 45% methane and 30-55% carbon dioxide and admixture of other gases, e.g. hydrogen sulfide, carbon monoxide, oxygen and nitrogen. Biogas net calorific value is within the limits of 17-27 MJ/Nm³ and depends mainly on the methane content. The net calorific value of pure methane is 35.8 MJ/Nm³ and the chemical energy content of 1m₃ of biogas is ca. 5.3 kWh.

Typical biogas plant is composed of the preliminary biomass tanks, fermentation tanks, covered with a tight membrane, post-fermentation tanks and cogeneration system (gas engine plus electrical generator) generating electric and heat energy from the refined biogas or biogas separated into methane and carbon dioxide, where methane is added to biogas which constitutes gas fuel. After reception and short storage in preliminary tanks a specific part of biomass is every day shredded, mixed with water and pumped out to fermentation tanks. In the tanks, on the average during 30-50 days from the delivery of the biomass portion, fermentation takes place and biogas is generated which accumulates in the upper part of the fermentation tank with a convex membrane maintaining specific gas pressure. Next, biogas is refined and delivered to the cogeneration system where it is combusted. Generated electric energy is sold to electric network and partly used for heating of fermentation tanks. The produced electric energy has the status of renewable energy.

An example of biogas plant is the installation presented in the Polish submission PL 393531. The installation consists of a two-chamber fermenter and digesting tank with separator. The biogas obtained is stored in a tank and delivered to cogenerator. This installation makes use of natural biogas and its efficiency is variable depending on the composition of the organic substrates which are subjected to methane fermentation during which biogas is generated with the methane content of 40 to 85%.

Increase of the net calorific value of biogas used in cogenerators is the goal of innovative technologies relating biogas enrichment with methane originating from the same biogas.

The Polish patent description PL 197595 presents the manner of generating methane and heat energy consisting of anaerobic biomass processing to biogas in separated processes of biomass hydrolysis and methane fermentation by mesophilic, thermophilic and psychrophilic methane bacteria found in returned wash. Purified biogas is separated into methane and carbon dioxide. From a part of methane and biogas, standard gas fuel is generated sued for the drive of the power generating unit and thermocontrol element generating electric energy and heat.

The Polish submission PL 349979 presents the manner of using the biogas containing methane, especially gas originating from waste dump and biogas from fermentation installations or disintegration processes in sewage treatment plants. Biogas is separated in the membrane separation installation into two gas streams. The first gas stream as compared to the biogas composition has a higher methane content and used as a combustible gas for powering gas engine. The second gas stream, enriched with CO₂ is returned to biogas generation process. This manner, similarly like the manner presented in PL 197595, consists of increasing the net calorific value only of a part of biogas generated in biogas plant because biogas is separated into two parts and the net calorific value of one part is enriched by using methane separated from the second part of biogas.

The device presented in patent description PL 197595 has a biomass preparation system connected with hydrolyzer and then with the serial fermenters system and compost bin cooperating with the return system and wash enrichment. Raw biogas tank is connected with the biogas purification system and further with the purified biogas tank connected with the biogas separation system and with gas mixer. The biogas separation system has outlets to CO₂ processing system and methane processing system also connected with the gas mixer, connected with the standard gas fuel tank. Fuel tank is connected with the electric and heat energy generation system and with the heat processing system.

It is well known - the biogas processing with addition of oxygen or water steam in plasma reactors such as for example the reactor presented in the patent description US 5993761 for obtaining of synthesis gas i.e. a mixture of carbon monoxide and hydrogen. Synthesis gas obtained in this way has the same net calorific value as the processed biogas and can be used especially for chemical syntheses.

DE 42 20 865 discloses a plasma reaction of CO2 and hydrogen to methane in order to produce a fuel gas. D1 does not disclose to react a mixture of CO2 and methane, as derived from a biogas, to increase the methane content by further hydrogenation of the CO2.

EP 2 169 035 discloses to increase the calorific value of a CH4/CO2 mixture by plasma treatment. The CH4 is reacted to hydrocarbons with C2-C4, which may by hydrogenated by H2 released from the cracked CH4. Surplus H2 is separated.

The manner of increasing net calorific value of biogas according to this invention consists of the following: the generated biogas is submitted to drying and next purification of sulfur compounds, after which it is compressed. Next, biogas is mixed with hydrogen, and mixed gases are hydrogenated in unbalanced plasma with the electron energy of ca. 1 eV and the inert gas temperature within the range of 1500K - 2000K, as a result of which carbon dioxide is hydrogenated and aliphatic hydrocarbons C1-C2 are created. Next, post-reaction gases are cooled down and hydrogen is separated and returned to plasma reactor, while the remaining gas mixture is combusted and energy is created, and the resulting combustion gases are dried and dust filtered. Next, nitric oxides are decomposed, after which carbon dioxide is separated, and the remaining gas, whose main component is nitrogen, is conducted to the chimney.

The device for increasing net calorific value of the biogas generated in biogas plant, according to this invention, has a drying system, biogas sulfur compounds purification system and purified gas compressing system and has a plasma reactor with the gas feeding system, equipped with post-reaction gases cooler which has a condensed water tank, and is connected with the hydrogen separator. Post-reaction gas cooler and hydrogen separator are connected with post-reaction gas control system. Hydrogen separator is connected with the gas feeding system and with the post-reaction gases control system and with cogenerator. Cogenerator is connected through the combustion gases drying system and dust filter with the reactor for NOₓ decomposition connected with the carbon dioxide separation system from the combustion gases. The hydrogen return line connects the hydrogen separator with the gas feeding system leading to plasma reactor. Carbon dioxide separation system separating carbon dioxide from combustion gases is connected with cogenerator by the carbon dioxide return line.

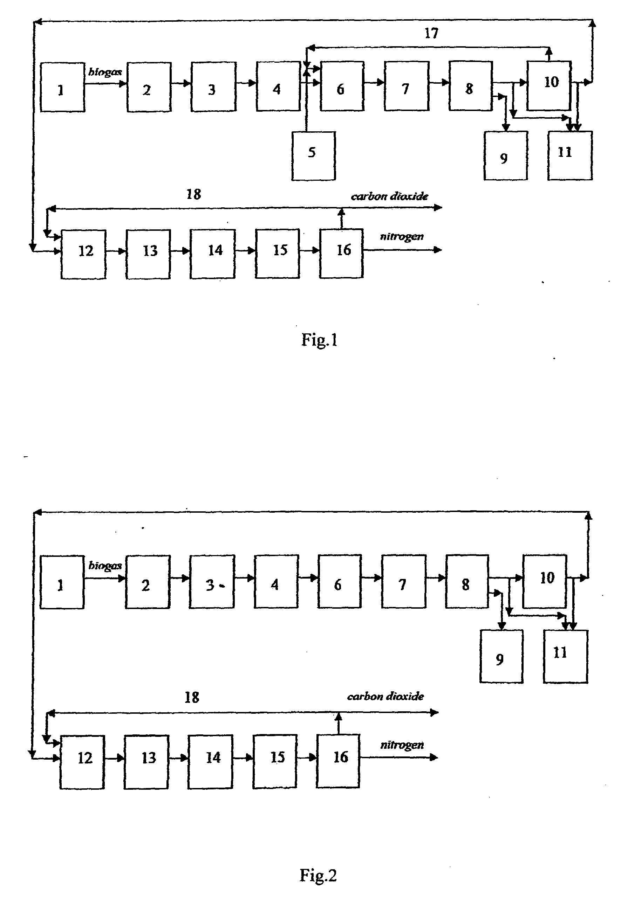

The subject of the invention is presented in the example of performance in the drawing, Fig. 1 presents the device version without hydrogen return, and Fig.2 the device version with the hydrogen return.

The device operation rule is as follows:
Biogas generated in biogas plant 1 is dried in the drying system **2** and then, purified of sulfur compounds in reactor **3**, after which it is compressed with the use of a compressor **4** and fed to gas feeding system **6**. Gas feeding system **6** to plasma reactor **7** is composed of a mixer and a nozzle. Biogas together with hydrogen is let into plasma reactor **7** where plasma is generated with electron energy of ca. 1 eV and inert gas temperature within the range of 1500K-2000K. Then, hydrogen contained in post-reaction gases is separated in hydrogen separator **10** and fed to gas feeding system **6** with the use of hydrogen return line **17** in the manner including control of its pressure and flow. Then, post-reaction gases are let to cogenerator **12**. Combustion gases from the cogenerator **12** are dried in the drying system **13**, dust is filtered in filter system **14** and the gases are let to reactor **15** where nitric oxides are decomposed. Then, carbon dioxide is separated from the combustion gases in the carbon dioxide separation system **16**, a part of which can be fed to cogeneator **12,** in the manner including control of its pressure and flow, with the use of the carbon dioxide return system **18.** The remaining gases, with the main component nitrogen, are let to the chimney.

During start-up of the device the biogas hydrogenation reaction is initiated by the hydrogen accumulated in tank **5** and fed in a controlled manner to gas feeding system **6**. It is also possible to carry out the process in such a way where carbon dioxide hydrogenation takes place on the basis of the hydrogen contained in methane. In such a case, hydrogen flow to gas feeding system **6** is cut off. This process is controlled by the control system **11** which monitors gases composition before and after the reaction. If necessary, hydrogen can be fed to plasma reactor **7** also during the hydrogenation process.

An advantage of this invention is the use of the plasma reactor which generates unbalanced plasma for the qualitative change of the gas fuel generated in biogas plant by increasing its net calorific value. As a result of the chemical reaction in the plasma generator which generates unbalanced plasma, carbon dioxide contained in biogas is hydrogenated and simple aliphatic hydrocarbons are created. In this way, carbon dioxide from biogas is transformed into components with high net calorific value. An additional advantage of use of plasma reactor is the possibility to control the initial parameters of the process within the entire scope of changes of biogas composition in the course of its enriching which enables to use the device according to the invention independently from the type of the biomass used.

Advantageous effects of the invention are the transformation of the carbon dioxide contained in biogas into simple aliphatic hydrocarbons which causes enriching of gas due to increase of its net calorific value and improvement of gas adequacy for use in gas engines through the possibility to control its composition relating the content level of CO₂. As a result, the processed biogas, in relation to the unprocessed gas, has much higher combustion heat, thus also net calorific value, which is shown in the examples below. Since the main component of chimney gas is nitrogen, the biogas enriching installation is environment friendly and the very process in practice produces no waste.

### Example 1

100 m³ of biogas composed of 45% vol. methane and 55% vol. carbon dioxide after drying and purification of sulfur compounds together with hydrogen was let to plasma reactor operating on the gliding discharge basis. Biogas enriching process was carried out for one hour. It turned out that post-reaction gases after leaving the plasma reactor and after hydrogen separation contained 12.7% CO₂, 71.9% CH₄, 12.8% C₂H₂ and 2.6% C₂H₄. Thus 62% of carbon contained in the input biogas were in post-reaction gases in the form of methane, 22% in the form of ethine, 4% in the form of ethene. While 11% of carbon remained in carbon dioxide. The net calorific value of post-reaction gases was 31 MJ/m³ (in reference to temperature 25°C). The net calorific value of output biogas was15 MJ/m³. After biogas enriching in plasma reactor, net calorific value of post-reaction gases increased by 110% of the net calorific value of the biogas used for enriching.

### Example II

100 m³ of biogas composed of 70% vol. methane and 30% vol. carbon dioxide after drying and purification of sulfur compounds together with hydrogen was let to plasma reactor operating on the gliding discharge basis. Biogas enriching process was carried out for one hour. It turned out that post-reaction gases after leaving the plasma reactor and after hydrogen separation contained 7% CO₂, 77.2% CH₄, 13.2% C₂H₂ and 2.7% C₂H₄. Thus 67% of carbon contained in the input biogas were in post-reaction gases in the form of methane, 23% in the form of ethine, 5% in the form of ethane, and 6% of carbon remained in carbon dioxide. The net calorific value of post-reaction gases was 33 MJ/m³ (in reference to temperature 25°C). The net calorific value of input biogas was 23 MJ/m³. After biogas enriching in plasma reactor, net calorific value of post-reaction gases increased by 43% of the net calorific value of the biogas used for enriching.

### Example III

100 m³ of biogas composed of 45% vol. methane and 55% vol. carbon dioxide after drying and purification of sulfur compounds together with hydrogen was let to plasma reactor operating on the gliding discharge basis. Biogas enriching process was carried out for one hour. It turned out that post-reaction gases after leaving the plasma reactor and after hydrogen separation contained 17.4% CO₂, 77.1% CH₄, 4.9% C₂H₂ and 0.6% C₂H₄. Thus 73% of carbon contained in the input biogas were in post-reaction gases in the form of methane, 9% in the form of ethine, 1% in the form of ethane, and 17% of carbon remained in carbon dioxide. The net calorific value of post-reaction gases was 28 MJ/m³ (in reference to temperature 25°C). The net calorific value of input biogas was 15 MJ/m³. After biogas enriching in plasma reactor, net calorific value of post-reaction gases increased by 89% of the net calorific value of the biogas used for enriching.

The efficiency of the process depends on the selection of the initial conditions of the process (flow, power, specific energy of gases) which is shown in examples I and III, in which various net calorific values were obtained with the same biogas composition. The process is controlled by monitoring the post-reaction gas composition and their technical specifications.

The device can be also applied in each biogas plant with observation of the size criterion where the increase of the biogas net calorific value is higher than the cost of the device installation and operation. Besides, the device can be used in every installation where it is required to enrich biogas with components of high net calorific value.

## Claims

1. The manner of increasing biogas net calorific value where the generated biogas is subjected to drying and next to purification of sulfur compounds and compression **characterized in that** biogas is mixed with hydrogen and the mixed gases are subjected to hydrogenation in unbalanced plasma with the electron energy of ca. 1 eV and inert gas temperature within the range of 1500K-2000K, as a result of which carbon dioxide is hydrogenated and aliphatic hydrocarbons C1-C2 are created, then post-reaction gases are cooled down and hydrogen is separated, while the remaining gases mixture is combusted and energy is generated, and the resultant combustion gases are dried, dust is filtered, and then nitric oxides are decomposed, after which carbon dioxide is separated fom combustion gases, and the resultant gas, with nitrogen as the main gas, is let out to chimney.

2. The device for increasing net calorific value of biogas equipped with the following systems connected with biogas plant: **/1/** drying system, **/2/** biogas purification system of sulfur compounds, **/3/** and purified gas compressing system, **/4/ characterized in that** it has a plasma reactor **/7/** with gas feeding system **/6/**, equipped with post-reaction gases cooler **/8/** with condensed water tank **/9/,** connected with hydrogen separator **/10/,** where the post-reaction gases cooler **/8/** and hydrogen separator **/10/** are connected with the post-reaction gases control system **/11/,** and hydrogen separator **/10/** is connected with hydrogen tank **/5/** connected with the gases feeding system **/6/** and post-reaction gases control system **/11/** and cogenerator **/12/**, which in turn, is connected through the combustion gases drying system **/13/** and dust filter **/14/** with the reactor used for NOₓ decomposition **/15/** connected with the carbon dioxide separation system from the combustion gases **/16/,** where the hydrogen separator **/10/** with the hydrogen tank **/5/** and gas feeding system **/6/** to plasma reactor **/7/** is connected by hydrogen return line **/17/**, and the carbon dioxide separation system from the combustion gases **/16/** is connected with cogenerator **/12/** by carbon dioxide return line **/18/**.

## Patentansprüche

1. Das Verfahren zur Steigerung des Brennwertes von Biogas, bei dem das erzeugte Biogas entfeuchtet wird und anschließend entschwefellt und verdichtet wird, **gekennzeichnet dadurch, dass** das Biogas mit Wasserstoff vermengt wird und das Gasgemisch der Hydrierung im Nichtgleichgewichtsplasma, mit Elektronenenergie von etwa 1eV und in Temperatur des Inertgases im Bereich von 1500K-2000K unterzogen wird, infolge wessen Kohlendioxid hydriert wird und aliphatische Kohlenwasserstoffe C1- C2 entstehen, dann werden die Nachreaktionsgase abgekühlt und der Wasserstoff wird abgesondert, das restliche Gasgemisch wird verbrannt, wobei Energie erzeugt wird; die entstandenen Abgase werden entfeuchtet, Staub wird abgefiltert, Stickoxide werden zersetzt und danach scheidet Kohlendioxid aus den Abgasen aus und das restliche Gas, dessen Hauptbestandteil Stickstoff ist, wird in den Schornstein abgeleitet.

2. Anlage zur Erhöhung des Brennwertes von Biogas ausgestattet mit: mit Biogasanlage /1/ verbundenes Entfeuchtungssystem /2/, Biogasentschwefelungssystem /3/ und Verdichtungssystem /4/des gereinigten Gases, **gekennzeichnet dadurch, dass** sie einen Plasmareaktor /7/ besitzt, mit Gaszufuhrsystem /6/, ausgestattet mit einem Kühlungssystem /8/ der Nachreaktionsgase mit Behälter /9/ für Kondenswasser, verbunden mit Wasserstoffabscheider /10/, wobei das Kühlungssystem der Nachreaktionsgase sowie Wasserstoffabscheider /10/, mit Kontrollsystem /11/ der Nachreaktionsgase und Wasserstoffabscheider /10/ mit Wasserstoffbehälter verbunden ist /5/, verbunden mit Gaszufuhrsystem sowie Kontrollsystem /11/ der Nachreaktionsgase und KWK-Anlage /12/ die wiederum durch Abgasentfeuchtungssystem /13/ und Staubfilter /14/ mit Reaktor für Zersetzung von Stickoxiden /15/ verbunden mit Abscheider /16/ von Kohlendioxid aus den Abgasen verbunden ist, wobei den Kohlenwasserstoffabscheider /10/ mit Kohlenwasserstoffbehälter /5/ und Gaszufuhrsystem /6/ in den Plasmareaktor /7/ die Wasserstoffrückführungslinie /17/ verbindet und den Abscheider von Kohlendioxid aus den Abgasen /16/ mit KWK-Anlage /12/ die Kohlendioxidrückführungslinie /18/ verbindet.

## Revendications

1. La méthode d'accroître le pouvoir calorifique du biogaz selon laquelle le biogaz fabriqué est sousmis à la déshumidification, et ensuite à la purification des composés du soufre et à la compression, **caractéristique par le fait** que le biogaz est mélangé à l'hydrogène et les gaz mélangés sont soumis à l'hydrogénation dans le plasma déséquilibré d'énergie d'électrons de rang de 1 eV et de température du gaz neutre de l'étendue de 1500K à 2000K, dont le résultat est l'hydrogénation du dioxide de carbone et la formation d'hydrocarbures aliphatiques C1-C2, ensuite les gaz étant le produit de la réation sont refroidis, l'hydrogène est séparé, et le mélange de gaz produit est soumis à la combustion avec la production d'énergie, tandis que les gaz de combustion produits sont soumis à la déshumidification, les poussières sont filtrées et ensuite les oxydes d'azote sont décomposés, suite à quoi le dioxyde de carbone se sépare des gaz de combustion et le gaz qui reste, dont le composant principal est l'azote est évacué dans la cheminée.

2. Le dispositif servant à accroître le pouvoir calorifique du biogaz est est équipé d'éléments suivants, raccordés à l'usine de biogaz (**1**): système (**2**) de déshumidification, système (**3**) de purification du biogaz des composés du soufre et système (**4**) de compression du gaz purifié, **se caractérisant par le fait qu'**il est muni de réacteur (**7**) plasmatique avec le système (**6**) d'amenée de gaz, équipé de radiateur (**8**) de gaz étant le produit de la réaction, avec le réservoir (**9**) d'eau condensé, raccordé au séparateur (**10**) d'hydrogène, étant donné que le radiateur (**8**) de gaz étant le produit de la réaction et le séparateur (**10**) d'hydrogène sont raccordés au système (**11**) de contrôle de gaz étant le produit de la réaction, et le séparateur (**10**) d'hydrogène est raccordé au réservoir (**5**) d'hydrogène raccordé au système (**6**) d'amenée de gaz et au système (**11**) de contrôle de gaz étant le produit de la réaction et au cogénérateur (**12**) qui à son tour est raccordé par l'intermédiaire du système (**13**) de déshumidification de gaz de combustion et du filtre (**14**) de poussières au réacteur (**15**) de décomposition de Nox raccordé au système (**16**) de séparation de dioxyde de carbone des gaz de combustion, étant donné que le séparateur (**10**) d'hydrogène est raccordé au réservoir (**5**) d'hydrogène et au système (**6**) d'amenée de gaz au réacteur (**7**) plasmatique par l'intermédiaire de la ligne (**17**) de retour d'hydrogène, et le système (**16**) de séparation du dioxyde de carbone des gaz de combustion est raccordé au cogénérateur (**12**) par l'intermédiaire de la ligne (**18**) de retour du dioxyde de carbone.
